**Europäisches Patentamt**

**European Patent Office**

**Office européen des brevets**

(11) Veröffentlichungsnummer: **0 212 310 B1**

(12) **EUROPÄISCHE PATENTSCHRIFT**

(45) Veröffentlichungstag der Patentschrift: **03.03.93**

(21) Anmeldenummer: **86110146.7**

(22) Anmeldetag: **23.07.86**

(51) Int. Cl.⁵: **A61K 31/10**

(54) Verwendung von Probucol zur Herstellung eines Arzneimittels für die Behandlung von Herzarrhythmie.

(30) Priorität: **23.08.85 DE 3530256**

(43) Veröffentlichungstag der Anmeldung:
**04.03.87 Patentblatt 87/10**

(45) Bekanntmachung des Hinweises auf die Patenterteilung:
**03.03.93 Patentblatt 93/09**

(84) Benannte Vertragsstaaten:
**AT BE CH DE FR GB IT LI LU NL SE**

(56) Entgegenhaltungen:

**AM. HEART J., Band 107, Nr. 4, 1984, Seiten 680-684; K.F. BROWNE et al.: "Prolongation of the OT interval induced by probucol: Demonstration of a method for determining OT interval change induced by a drug"**

**ANN. N.Y. ACAD. SCI. (USA), Band 427, 1984, Seiten 307-318; D.P. ZIPES et al.: "Treatment of patients with life-threatening cardiac arrhythmias"**

(73) Patentinhaber: **MERRELL DOW PHARMACEUTICALS INC.**
**2110 East Galbraith Road**
**Cincinnati Ohio 45215-6300(US)**

(72) Erfinder: **Schwartzkopff, Wolfgang, Prof. Dr. med.**
**Xantener Strasse 18**
**W-1000 Berlin 15(DE)**
Erfinder: **von Leitner, Enz-Rüdiger, Prof. Dr. med.**
**Städt. Krankenhaus Siloah Rösebeckstrasse 15**
**W-3000 Hannover 91(DE)**

(74) Vertreter: **Vossius & Partner**
**Siebertstrasse 4 P.O. Box 86 07 67**
**W-8000 München 86 (DE)**

EP 0 212 310 B1

EUROPEAN JOURNAL OF CLINICAL PHAR-
MACOLOGY, Band 26, Nr. 6, 1984, Seiten
735-739, Springer-Verlag; C.A. DUJOVNE et
al.: "Electrocardiographic effects of probucol. A controlled prospective clinical trial"

ARTERY, Band 10, Nr. 1, 1982, Seiten 56-70;
D. McCAUGHAN et al.: "Nine years of treatment with probucol"

THE LANCET, 12. Juni 1982, Seite 1365; B.L.
MARTZ: "Probucol and the OT interval"

Deutsches Ärzteblatt, Heft 27, 05.07.90. B-
1524-25, Lüderitz B.

**Beschreibung**

Die Erfindung betrifft die Verwendung von Probucol zur Herstellung eines Arzneimittels für die Behandlung von Herzarrhythmie.

Probucol wird in einer Dosierung von 1000 mg/Tag zur Behandlung von bestimmten Stoffwechselstörungen verwendet, bei denen der Blutcholesterinspiegel erhöht ist; vgl. US-A 38 62 332. Überraschend wurde gefunden, daß Probucol eine antiarrhythmische Wirkung besitzt. Nach der Klassifizierung der Antiarrhythmika nach VAUGHN-WILLIAMS ist Probucol ein Antiarrhytmikum der Gruppe III. Einzige bekannte Vertreter dieser Gruppe sind Amiodaron und Sotalol. Beide Substanzen besitzen erhebliche Nebenwirkungen.

Im Rahmen einer Pilotstudie wurde das Arrhythmieverhalten und die QT-Zeit systematisch bei Patienten mit Fettstoffwechselstörungen untersucht, die im Rahmen einer kontrollierten Studie mit Probucol behandelt wurden.

Patienten und Methode:

Bei 42 Patienten mit Hyperlipoproteinämie wurde im Rahmen einer kontrollierten Studie die Wirkung von Probucol gegen Placebo geprüft.

I. Standard-EKG

Je ein 12-Ableitungs-Standard-EKG wurde registriert.
1. In der Placebophase
2. In der Verumphase nach 12 Wochen
3. In der Verumphase nach 24 Wochen
4. In der Placebophase 42 Tage nach Absetzen von Probucol
Für jede der Aufzeichnungen wurde jeweils die frequenzkorrigierte QT-Zeit ermittelt (Bazett-Formula).

Statistische Berechnung:

Unter Verwendung des Wilcoxon-Tests für verbundene Stichproben wurde geprüft, ob ein signifikanter Unterschied der QT-Dauer zwischen den Placebophasen und den Verumphasen besteht.

II. 24-Stunden-Langzeit-EKG:

Von allen 42 Patienten wurde mittels Oxfolg-Medilog II Registriergeräten ein 24-Stunden-Langzeit-EKG sowohl in der Verum- als auch in der Placebophase registriert.

Die Bänder wurden ohne Kenntnis der Therapie unter Verwendung eines Reynolds Pathfinder-Analysesytems ausgewertet, dessen Sensitivität zur Identifikation von QRS-Komplexion bei 99 %, zur Erkennung ventrikulärer Extrasystolen bei 96 % lag. Neben den normalen und extrasystolischen QRS-Komplexen wurden auch die folgenden Arrhythmieformen quantitativ erfaßt: supraventrikuläre Extrasystolen, supraventrikuläre Tachykardien, ventrikuläre Extrasystolen, R/T-Phänomene, gepaarte ventrikuläre Extrasystolen, mehrere hintereinander auftretende ventrikuläre Extrasystolen, Kammertachykardien, ventrikuläre Bigemini und Asystolien. Darüber hinaus wurden erfaßt Phasen intermittierenden Vorhofflimmerns, intermittierende Schenkelblockbilder und das Herzfrequenzverhalten.

Die stündliche Häufigkeit der verschiedenen Rhythmusstörungen wurden erfaßt und auf einem Computerausdruck dokumentiert. Die Analyse wurde korrigiert, indem der Vorgang jedes Mal unterbrochen wurde, wenn eine ventrikuläre Extrasystole erkannt wurde. Diese wurde dann jeweils 1 : 1 auf EKG-Papier ausgeschrieben und durch den Untersucher verifiziert. Im Falle der falsch positiven Erkennung eines Ereignisses wurde der Computerausdruck entsprechend korrigiert. Dadurch wurde die positive Korrektheit der Analyse auf 100 % gesteigert. In Tabelle 3 sind die korrigierten Ergebnisse wiedergegeben.

Bei der relativen Seltenheit komplexer tachykarder ventrikulärer Rhythmusstörungen im untersuchten Kollektiv wurde eine Bewertung des Therapieeffektes lediglich für die ventrikulären Extrasystolen vorgenommen. Hierbei ließ sich die Frage nach einem möglichen paradoxen arrhythmogenen Effekt naturgemäß für alle Patienten beantworten, die Frage nach einem antiarrhythmischen Effekt dagegen nur bei den Patienten, deren spontane Arrhythmiehäufigkeit in der Placebophase eine gewisse Häufigkeit überschritt. Hierfür wurde willkürlich eine Grenze von wenigstens einer Extrasystole/Stunde, entsprechend 24 in 24 Stunden, gefordert.

Als Hinweis auf einen möglichen paradoxen arrhythmogenen Effekt des Medikaments werteten wir eine Zunahme der Extrasystoliehäufigkeit gegenüber Placebo um wenigstens 150 %. Als Hinweis auf einen möglichen antiarrhythmischen Effekt werteten wir eine Abnahme der spontanen Extrasystoliehäufigkeit um wenigstens 60 %, als Hinweise auf einen wahrscheinlichen antiarrhythmischen Effekt eine Abnahme der Extrasystoliehäufigkeit um wenigstens 75 %.

Ergebnisse:

I. QT-Zeit (Tabelle 1 und 2)

Während der 1. Placebophase betrug die mittlere QT-Zeit 392 ± 27 ms, nach 12-wöchiger Therapie mit Probucol 411 ± 29 s, nach 24-wöchiger Therapie mit Probucol 412 ± 27 s, in der abschließenden Placebophase 402 ± 25 s. Die Werte der QT-Zeit während der beiden Therapiephasen unterschieden sich nicht signifikant voneinander ($p = 0.753$). Dagegen waren die QT-Zeiten in den beiden Placebophasen signifikant unterschiedlich ($p < 0.018$). Wir erklärten dies durch eine noch vorhandene Wirkung des Präparates 42 Tage nach dem Absetzen infolge der langen Halbwertszeit von Probucol.

Die frequenzkorrigierte QT-Zeit unter Probucol (Therapiephase 1 bzw. 2) war im Vergleich zu den Placebophasen 1 bzw. 2 signifikant verlängert ($p = 0.002$). Ebenfalls ein hochsignifikanter Unterschied fand sich beim Vergleich der QT-Dauer während der 1. Placebophase mit der mittleren QT-Dauer der beiden Verumphasen (p 0.001). Eine Verlängerung der QT-Zeit impliziert jedoch keine antiarrhythmische Wirkung.

II. Arrhythmieverhalten (Tabelle 3)

84 Langzeit-EKG-Bänder wurden bislang analysiert. Zwei Aufzeichnungen waren quantitativ nicht analysierbar wegen rezidivierend auftretender idioventrikulärer Tachykardien mit Interferenzdissoziation sowohl unter Placebo als auch unter Verum. Sieben weitere Registrierungen müssen wiederholt werden, da entweder eine zu starke Artefaktüberlagerung vorlag und/oder sonstige Probleme eine einwandfreie quantitative Analyse der Bänder unmöglich machten. Damit liegen zur Beurteilung der Frage, ob Probucol eine paradoxe arrhythmogene Wirkung entfalte, bislang 64 Registrierungen von 32 Patienten vor. Von diesen 32 Patienten zeigten 3 unter Probucol eine signifikante Zunahme der spontanen Arrhythmiehäufigkeit. Dies entspricht dem Verhalten der gebräuchlichen Antiarrhythmika, die ebenfalls eine paradoxe arrhythmogene Wirkung von 10 bis 15 % der Fälle entfalten.

Ein ventrikulärer Bigeminus trat bei 2 Patienten unter Placebo auf, jedoch nicht unter Verum, dagegen bei einem Patienten unter Verum, jedoch nicht unter Placebo. Gepaarte oder salvenförmig einfallende ventrikuläre Extrasystolen traten zu selten auf, um hier eine Wertung vornehmen zu können.

17 Patienten zeigten unter Placebo eine Häufigkeit der ventrikulären Extrasystolen, welche im 24-Stunden-Mittel mehr als 1 VES/h betrug. Bei 7 dieser Patienten betrug die Reduktion der Extrasystolen unter Therapie über 75 %, bei 3 weiteren lag sie zwischen 60 und 75 %. Damit ist bei 59 % der Patienten eine Reduktion der spontanen Extrasystoliefrequenz beobachtet worden, die auf eine antiarrhythmische Wirkung von Probucol hinweist. Der Wert dieser Aussage wird allerdings eingeschränkt durch die insgesamt geringe Arrhythmiehäufigkeit im untersuchten Patientenkolletiv. Die Aussage wird um so sicherer, je größer die spontane Arrhythmiehäufigkeit ist. Betrachtet man getrennt lediglich die Patienten, deren spontane Arrhythmiehäufigkeit im 24-Stunden-Mittel über 15 VES/h beträgt, so handelt es sich hierbei um 8 Patienten. Von diesen zeigen allerdings unter Therapie 5 eine statistisch signifikante Abnahme der Extrasystoliefrequenz um mehr als 75 % (89, 90, 94, 95 bzw. 100 %). Drei weitere zeigen eine Abnahme der Extrasystolie um wenigstens 60 % (60, 66 bzw. 69 %). Kein einziger dieser Patienten, bei denen eine Aussage doch mit großer Sicherheit gemacht werden kann, zeigt eine völlig fehlende Wirkung oder gar eine paradoxe arrhythmogene Wirkung.

Zusammenfassende Beurteilung:

Bei 41 Patienten mit Hyperlipoproteinämie wurde eine placebokontrollierte Behandlung mit Probucol durchgeführt.

Die während der Placebo- und Verum-Phasen registrierten 12-Ableitungs-EKG belegen, daß Probucol in einer Dosierung von 1000 mg täglich zu einer signifikanten Verlängerung der frequenzkontrollierten QT-Zeit um ca. 5 % führt. Gleichzeitig finden sich bei 59 % der Patienten, die eine spontane Häufigkeit ventrikulärer Extrasystolen von wenigstens 24 in 24 Stunden haben, Hinweise auf eine antiarrhythmische Wirkung des Medikaments. Diese läßt sich um so deutlicher nachweisen, je größer die spontane Arrhythmiehäufigkeit ist.

Dies erklärt sich dadurch, daß bei diesen Patienten mit einer geringeren Spontanvariabilität gerechnet werden kann. Bei geringer Spontanarrhythmiehäufigkeit verschleiert dagegen die Spontanvariabilität häufig einen möglicherweise vorhandenen antiarrhythmischen Effekt. Bei 8 Patienten, deren spontane Arrhythmiehäufigkeit in dem Bereich liegt, der eine sichere Aussage über einen möglichen antiarrhythmischen Effekt des Präparates erlaubt, ließ sich fünfmal ein eindeutiger antiarrhythmischer Effekt sichern, weitere dreimal eine solche Wirkung wahrscheinlich machen.

Bei 3 von 32 Patienten wurde eine paradoxe arrhythmogene Wirkung beobachtet im Sinne einer Zunahme der spontanen Arrhythmiehäufigkeit. Allerdings betrug in allen 3 Fällen die Extrasystoliefrequenz unter Placebo weniger als 3 VES/ h, so daß die Zunahme der Extrasystolen möglicherweise zum Teil Folge der Spontanvariabilität war. Bei keinem der 3 Patienten wurden komplexe tachykarde ventrikuläre Rhythmusstörungen unter Therapie beobachtet. Als Nebenwirkungen wurden gelegentlich Durchfall, Bauchschmerzen und Kopfschmerzen beobachtet.

Zusammenfassend sprechen die Befunde der vorliegenden Pilotstudie für eine antiarrhythmische Wirkung des Präparates Probucol im Sinne einer Unterdrückung der ventrikulären Extrasystolie. Die Wirkung scheint besonders ausgeprägt zu sein bei den Patienten, die spontan häufige Extrasystolen hatten.

Arzneimittel, die Probucol als Wirkstoff enthalten, können auf die in der US-A 38 62 332 beschriebene Weise hergestellt werden. Die Arzneimittel können oral oder parenteral, vorzugsweise oral, appliziert werden. Typische Beispiele für Darreichungsformen sind Tabletten und Injektionspräparate. Die Dosierung hängt ab von der Schwere der Erkrankung, Alter und Gewicht des Patienten. Die Tagesdosis kann etwa 2 bzw. 20 mg, vorzugsweise 4 bis 15 mg Wirkstoff pro Kilogramm Körpergewicht betragen.

## Tabelle 1

### QT-Zeit unter Verum bzw. Placebo

Probucol

QTc

| nach Ph. | Placebo F | Placebo QT | Verum-2 F | Verum-2 QT | Verum-4 F | Verum-4 QT | Placebo F | Placebo QT |
|---|---|---|---|---|---|---|---|---|
| Pat.-Nr. | | | | | | | | |
| 11 | 66 | 0,37 | 64 | 0,40 | 71 | 0,41 | 59 | 0,42 |
| 12 | 83 | 0,34 | 85 | 0,35 | 77 | 0,34 | 100 | 0,32 |
| 13 | 60 | 0,42 | 50 | 0,44 | 48 | 0,44 | 51 | 0,42 |
| 14 | 71 | 0,36 | 80 | 0,36 | 69 | 0,37 | 81 | 9,35 |
| 15 | 75 | 0,37 | 60 | 0,38 | 70 | 0,38 | 54 | 0,42 |
| 16 | | | | | | | | |
| 17 | 76 | 0,38 | 77 | 0,36 | 80 | 0,34 | 78 | 0,34 |
| 18 | 64 | 0,38 | 87 | 0,40 | 64 | 0,40 | 60 | 0,38 |
| 19 | 56 | 0,42 | 60 | 0,43 | 59 | 0,42 | 85 | 0,38 |
| 20 | 74 | 0,36 | 68 | 0,38 | 75 | 0,36 | 88 | 0,34 |
| 21 | 103 | 0,30 | 90 | 0,38 | 84 | 0,36 | 84 | 0,36 |
| 22 | 65 | 0,39 | 62 | 0,40 | 75 | 0,34 | 75 | 0,36 |
| 23 | | | | | | | | |
| 24 | 51 | 0,40 | 66 | 0,40 | 48 | 0,45 | 53 | 0,40 |
| 25 | 65 | 0,30 | 66 | 0,38 | 70 | 0,40 | 64 | 0,36 |

EP 0 212 310 B1

EP 0 212 310 B1

## Tabelle 1 - Fortsetzung

| nach Ph. | Placebo F | Placebo QT | Verum-2 F | Verum-2 QT | Verum-4 F | Verum-4 QT | Placebo F | Placebo QT |
|---|---|---|---|---|---|---|---|---|
| Pat.-Nr. | | | | | | | | |
| 26 | 50 | 0,42 | 59 | 0,42 | 53 | 0,44 | 51 | 0,43 |
| 27 | 61 | 0,41 | 58 | 0,44 | 55 | 0,48 | 77 | 0,37 |
| 28 | 66 | 0,38 | 65 | 0,40 | 60 | 0,38 | 59 | 0,39 |
| 29 | 60 | 0,40 | 56 | 0,43 | 61 | 0,43 | 61 | 0,43 |
| 30 | 50 | 0,42 | 49 | 0,44 | 48 | 0,42 | 52 | 0,44 |
| 31 | | | | | | | | |
| 32 | 47 | 0,44 | 51 | 0,42 | 57 | 0,41 | 62 | 0,40 |
| 33 | 100 | 0,32 | 66 | 0,40 | 55 | 0,44 | 52 | 0,43 |
| 34 | 60 | 0,38 | 65 | 0,42 | 58 | 0,40 | 63 | 0,42 |
| 51 | 52 | 0,39 | 51 | 0,42 | 61 | 0,40 | 52 | 0,40 |
| 52 | 88 | 0,32 | 75 | 0,34 | 74 | 0,34 | 71 | 0,35 |
| 53 | 64 | 0,40 | 69 | 0,41 | 69 | 0,40 | 76 | 0,38 |
| 54 | 60 | 0,41 | 61 | 0,40 | 65 | 0,40 | 66 | 0,41 |
| 55 | 68 | 0,32 | 68 | 0,39 | 85 | 0,40 | 76 | 0,36 |
| 56 | 65 | 0,35 | 58 | 0,38 | 57 | 0,39 | 58 | 0,37 |
| 57 | 65 | 0,38 | 66 | 0,41 | 62 | 0,40 | 80 | 0,37 |
| 58 | 57 | 0,41 | 60 | 0,40 | 76 | 0,40 | 61 | 0,41 |
| 59 | 62 | 0,40 | 59 | 0,39 | 66 | 0,40 | 54 | 0,41 |
| 60 | | | | | | | | |

7

EP 0 212 310 B1

Tabelle 1 - Fortsetzung

| nach Ph. | Placebo F | Placebo QT | Verum-2 F | Verum-2 QT | Verum-4 F | Verum-4 QT | Placebo F | Placebo QT |
|---|---|---|---|---|---|---|---|---|
| Pat.Nr. | | | | | | | | |
| 61 | 80 | 0,34 | 70 | 0,38 | 100 | 0,33 | 67 | 0,38 |
| 62 | 65 | 0,38 | 63 | 0,50 | 62 | 0,37 | 64 | 0,37 |
| 63 | | | | | | | | |
| 64 | | | | | | | | |
| 65 | 61 | 0,38 | 68 | 0,32 | 76 | 0,34 | 68 | 0,34 |
| 66 | 52 | 0,34 | 58 | 0,35 | 55 | 0,39 | 68 | 0,34 |
| 67 | 67 | 0,41 | 60 | 0,40 | 67 | 0,41 | 68 | 0,41 |
| 68 | | | | | | | | |
| 69 | 74 | 0,38 | 68 | 0,41 | 71 | 0,41 | 88 | 0,38 |
| 70 | 64 | 0,40 | 68 | 0,40 | 64 | 0,41 | 63 | 0,38 |
| 71 | 64 | 0,34 | 68 | 0,41 | 73 | 0,35 | 70 | 0,35 |
| 72 | 70 | 0,39 | 67 | 0,40 | 53 | 0,40 | 79 | 0,35 |
| 73 | 71 | 0,36 | 63 | 0,40 | 87 | 0,40 | 71 | 0,36 |
| 74 | 60 | 0,38 | 57 | 0,40 | 59 | 0,40 | 72 | 0,35 |
| N | 41,00 | 41,00 | 41,00 | 41,00 | 41,00 | 41,00 | 41,00 | 41,00 |
| Mittelw. | 66,39 | 0,38 | 64,32 | 0,40 | 66,49 | 0,38 | 67,83 | 0,38 |
| Standabw. | 12,44 | 0,03 | 8,57 | 0,03 | 11,33 | 0,03 | 11,35 | 0,03 |

## Tabelle 2

### Statistische Berechnung der Unterschiede der QT-Zeit unter Placebo bzw. Verum

| $QT_c$ | Placebo 1 | Verum 1 | Verum 2 | Placebo 2 |
|---|---|---|---|---|
| Placebo mg 1 | − | Z= −3,174 p <0,002 | Z= −3,174 p <0,002 | Z= −2,364 p <0,018 |
| Verum 1 | − | − | Z= −0,314 p= 0,753 | − |
| {Verum 1+2 2 mg | Z= −3,651 p <0,001 | − | − | − |

## Tabelle 3

Spontane Arrhythmiehäufigkeit unter Kontrolle und unter Therapie mit 1000 mg Probucol täglich

| | | KONTROLLE | | | THERAPIE | | | | | |
| | | | | | V E S | | V P | | V T | |
| Nr. | Std. | VES | VP | VS | $\Delta$ % | n/Std. | $\Delta$% | n/Std. | $\Delta$% | n/Std. |
|---|---|---|---|---|---|---|---|---|---|---|
| 11 | /24 | | | | – | – | | – | | – |
| 12 | 24/24 | – | – | – | + | 3 | | – | | – |
| 13 | 24/ | – | – | – | | | | | | |
| 15 | 24/24 | 18 | – | – | –90 | 2 | | – | | – |
| 16 | 24/24 | 19 | – | – | –68 | 6 | | – | | – |
| 17 | 24/23 | 12 | – | – | +17 | 14 | | 0,09 | | – |
| 18 | 24/24 | 0,5 | – | – | (–100) | – | | – | | – |
| 19 | 23/24 | 0,39 | – | – | (–100) | – | | – | | – |
| 20 | 24/ | 0,3 | – | – | D R O P – O U T | | | | | |
| 21 | 24/24 | 9 | – | – | +320 | 37,8 | | – | | – |
| 22 | /24 | | | | | 0,8 | | – | | – |
| 23 | 24/24 | 4 | – | – | –95 | 0,2 | | – | | – |
| 24 | 22/22 | 0,3 | – | – | (+) | 0,5 | | – | | – |
| 25 | 24/24 | 0,1 | – | – | + | 2 | | – | | – |
| 26 | 23/23 | 63,5 | – | – | –66 | 21,6 | | – | | – | Big./ |
| 27 | /24 | | | | | 30 | | 0,04 | | – | /Schen |
| 28 | 24/24 | 1 | – | – | –100 | – | | – | | – |

EP 0 212 310 B1

Tabelle 3 - Fortsetzung

| Nr. | Std. | KONTROLLE | | | V E S | | V P | | V T | |
| | | VES | VP | VS | Δ % | n/Std. | Δ % | n/Std. | Δ % | n/Std. |
|---|---|---|---|---|---|---|---|---|---|---|
| 29 | 24/23 | 0,6 | – | – | +8566 | 52 | | – | | – |
| 30 | 24/ | 0,3 | – | – | | | | | | |
| 31 | 24/24 | | IDIOVENTR. | TACHYKARDIE MIT INTERFERENZDISSOZIATION ! | | | | | | |
| 32 | 24/24 | 12,3 | – | – | +66 | 20 | | 0,1 | – | /Big |
| 33 | 24/24 | – | – | – | – | | | – | – | |
| 34 | 24/24 | 2 | – | – | –85 | 0,3 | | – | – | |
| 51 | /24 | | | | | 4 | | – | – | |
| 52 | 23/24 | 0,1 | – | – | = | 0,1 | | – | – | |
| 53 | 23/24 | 21 | – | – | –100 | – | | – | – | |
| 54 | | | | | | | | | | |
| 55 | 24/24 | 48 | – | – | –60 | 21 | | – | – | |
| 56 | /24 | | | | | – | | – | – | |
| 57 | 24/24 | – | – | – | = | – | | – | – | |
| 58 | 24/24 | – | – | – | = | – | | – | – | |
| 59 | | | | | | | | | | |
| 60 | | | | | | | | | | |
| 61 | 24/24 | 4 | – | – | –25 | 3 | | – | – | |
| 62 | 25/24 | 2,3 | – | – | +47 | 3,4 | | – | – | |
| 63 | | | | | | | | | | |
| 64 | | | | | | | | | | |

Tabelle 3 – Fortsetzung

| Nr. | Std. | KONTROLLE | | | THERAPIE | | | | | |
|---|---|---|---|---|---|---|---|---|---|---|
| | | VES | VP | VS | V E S Δ% | n/std. | V P Δ% | n/std. | V T Δ% | n/std. |
| 65 | 24/23 | 32 | 0,1 | – | –89 | 3,5 | | – | | – |
| 66 | 24/24 | 0,04 | – | – | (+) | 0,3 | | – | | – |
| 67 | 24/24 | 0,3 | – | – | (+) | 0,5 | | – | | 0,04 |
| 68 | 24/24 | 0,1 | – | – | (–100) | – | | – | | – |
| 69 | 24/23 | – | – | – | = | – | | – | | – |
| 70 | 24/ | 12 | – | – | | – | | | | – |
| 71 | 24/24 | – | – | – | | – | | – | | – |
| 72 | 24/23 | 72 | – | – | –94 | 4 | | – | | – |
| 73 | 24/24 | 0,4 | – | – | (+) | 0,9 | | – | | – |
| 74 | 24/24 | | | | | 13 | | | | – |
| 75 | /22 | | | | | | | | | |

## Patentansprüche

1.  Verwendung von Probucol zur Herstellung eines Arzneimittels für die Behandlung von Herzarrhythmie.

**Claims**

1.    Use of probucol for producing a drug for the treatment of cardiac arrhythmias.

**Revendications**

1.    Utilisation du Probucol pour la préparation d'un médicament pour le traitement de l'arythmie cardiaque.